# EUROPEAN PATENT APPLICATION

(11) **EP 4 691 344 A1**
(43) Date of publication of application: **11.02.2026**
(21) Application number: 24779767.3
(22) Date of filing: 19.03.2024
(51) Int. Cl.: A61B 3/103

(54) **OPHTHALMIC DEVICE AND OPHTHALMIC DEVICE CONTROL PROGRAM**

(30) Priority: 31.03.2023 JP 2023058641
(71) Applicant: NIDEK CO., LTD., Gamagori-shi, Aichi 443-0038 (JP)
(72) Inventor: SUZUKI Kunio, Gamagori-shi Aichi 443-0038 (JP); NAKAMURA Kenji, Gamagori-shi Aichi 443-0038 (JP); HAMAGUCHI Koji, Gamagori-shi Aichi 443-0038 (JP)
(74) Representative: Hoefer & Partner Patentanwälte mbB
(86) International application number: PCT/JP2024/010814
(87) International publication number: WO 2024/203642

(57) **Abstract**

The present invention addresses the technical problem of providing: an ophthalmic device that can suitably detect fundus-reflected light; and an ophthalmic device control program. Provided is an ophthalmic device for measuring eye characteristics of an eye under examination, said ophthalmic device characterized by comprising: a measurement optical system that has a measurement light source and an imaging element, and that measures said eye characteristics by performing imaging with the imaging element, using, as a pattern image, fundus-reflected light of measurement light emitted from the measurement light source; a focus adjustment means that moves a focus position of the imaging element in the optical axis direction of the imaging element; and a control means that controls the focus adjustment means to move the focus position if the pattern image is not detected from a measurement image obtained with the imaging element.

## Description

### TECHNICAL FIELD

The present disclosure relates to an ophthalmic apparatus that measures an eye characteristic of a subject eye, and an ophthalmic apparatus control program.

### BACKGROUND ART

In a related art, there has been known an eye refraction measurement apparatus that measures an eye characteristic of a subject eye by irradiating the subject eye with measurement light and detecting fundus reflection, or a wavefront aberration measurement apparatus.

### CITATION LIST

### PATENT LITERATURE

Patent Literature 1: JP2007-282671A

### SUMMARY OF INVENTION

In an ophthalmic apparatus of the related art, when an intermediate light projection body (for example, a cornea, a vitreous body, or a crystalline lens) is turbid due to intermediate light projection body opacity (for example, cataract), fundus reflection light cannot be detected, or internal reflection light of the apparatus is erroneously detected as the fundus reflection light, so that measurement cannot be performed well in some cases.

In view of the problem in the related art, a technical object of the present disclosure is to provide an ophthalmic apparatus and an ophthalmic apparatus control program capable of suitably detecting fundus reflection light.

In order to solve the above problem, the present disclosure includes the following configurations.
(1) According to an aspect of the present disclosure, there is provided an ophthalmic apparatus that measures an eye characteristic of a subject eye, the ophthalmic apparatus having:
   a measurement optical system that includes a measurement light source and an imaging element, and measures the eye characteristic by capturing, as a pattern image, fundus reflection light of measurement light emitted from the measurement light source using the imaging element;
   a focus adjustment unit that moves a focus position of the imaging element in an optical axis direction of the imaging element; and
   a control unit that controls the focus adjustment unit to move the focus position, in a case where the pattern image is not detected from a measurement image acquired by the imaging element.
(2) According to another aspect of the present disclosure, there is provided an ophthalmic apparatus control program executed in an ophthalmic apparatus that measures an eye characteristic of a subject eye, the ophthalmic apparatus control program comprising instructions which, when executed by a control unit of the ophthalmic apparatus, cause the ophthalmic apparatus to perform:
   a measurement step of measuring the eye characteristic by capturing, as a pattern image, fundus reflection light of measurement light emitted from a measurement light source using an imaging element; and
   a focus adjustment step of moving a focus position of the imaging element in an optical axis direction of the imaging element, in a case where the pattern image is not detected from a measurement image acquired by the imaging element.

According to the present disclosure, the fundus reflection light can be suitably detected.

### BRIEF DESCRIPTION OF DRAWINGS

[FIG. 1] FIG. 1 is a diagram showing an external configuration of a present example.
[FIG. 2] FIG. 2 is a diagram showing an internal configuration of the present example.

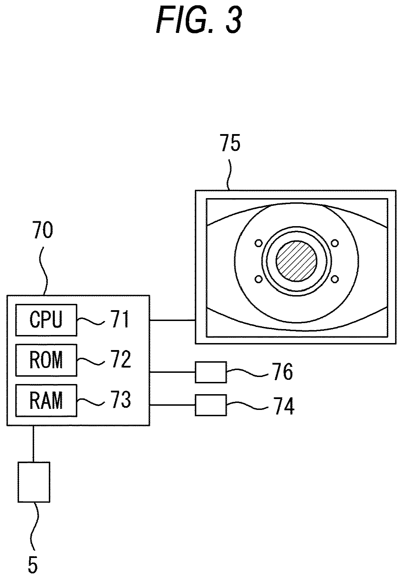

[FIG. 3] FIG. 3 shows a control system according to the present example.
[FIG. 4] FIG. 4 is a flowchart showing a control operation of the present example.
[FIG. 5A] FIG. 5A shows a measurement image of the present example.
[FIG. 5B] FIG. 5B shows a measurement image of the present example.
[FIG. 6A] FIG. 6A shows a measurement image of the present example.
[FIG. 6B] FIG. 6B shows a measurement image of the present example.

### DESCRIPTION OF EMBODIMENTS

### <Example>

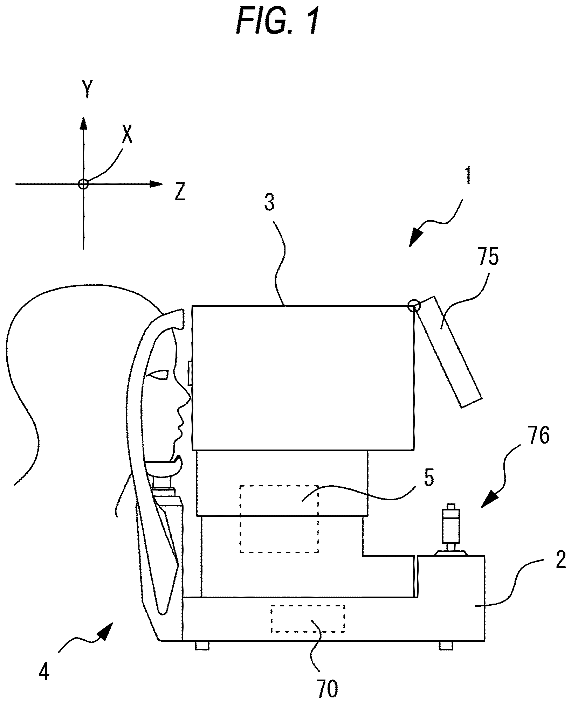

An example of the present disclosure will be described with reference to the drawings. An ophthalmic apparatus of the present example measures an eye refractive power of an examinee. As illustrated in FIG. 1, for example, the ophthalmic apparatus 1 includes a base 2, a measurement unit 3, a face support portion 4, a display unit 75, an operation unit 76, a drive unit 5, a control unit 70, and the like. The base 2 supports the measurement unit 3. The measurement unit 3 includes an optical system used for measuring a subject eye. The face support portion 4 is used to fix a face of the examinee in front of the measurement unit 3. The face support portion 4 is fixed to the base 2 and supports the face of the examinee. The drive unit 5 three-dimensionally moves the measurement unit 3 with respect to the base 2. The display unit 75 displays the eye refractive power and the like of the subject eye on a screen. The display unit 75 may function as a touch panel also serving as an operation unit. The operation unit 76 receives an operation from an examiner.

### [Measurement Unit]

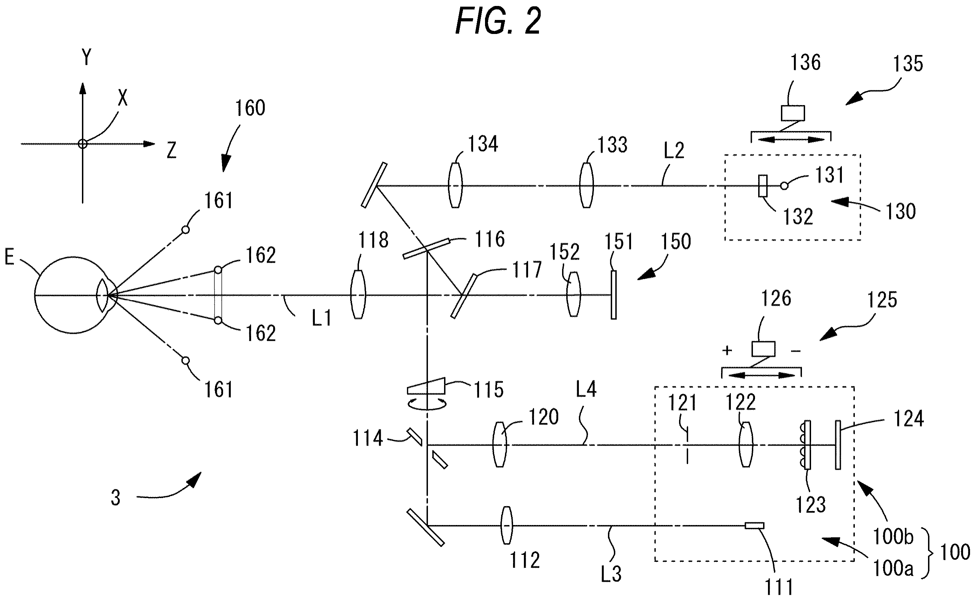

The optical system provided in the measurement unit 3 will be described with reference to FIG. 2. The measurement unit 3 includes, for example, a measurement optical system 100, a fixation target optical system 130, an observation optical system 150, and an index optical system 160. The measurement unit 3 further includes half mirrors 116, 117 that branch and couple optical paths of the respective optical systems, an objective lens 118, a focus adjustment unit 125 that drives a part of the optical system, a fixation target adjustment unit 135, and the like. In each optical system, the light source side is upstream, and the subject eye side is downstream.

### (Measurement Optical System)

The measurement optical system 100 is used to objectively measure the eye refractive power of the subject eye E. For example, each value of SPH: spherical power, CYL: cylindrical power, and AXIS: astigmatic axis angle is acquired as a measurement result of the eye refractive power. The measurement optical system 100 includes a light projection optical system 100a and a light reception optical system 100b.

The light projection optical system 100a includes a measurement light source 111 and a relay lens 112, and projects spot-shaped measurement light onto a fundus of the subject eye E via the central portion of a pupil or a corneal apex of the subject eye E. The measurement light source 111 may be an SLD light source, an LED light source, or another light source. In the present example, infrared light is used as the measurement light.

In the present example, a prism 115 is disposed on a common path of the light projection optical system 100a and the light reception optical system 100b. By rotating the prism 115 around an optical axis, a projection light flux on the pupil is eccentrically rotated at a high speed. As an example, in the present example, the projection light flux is eccentrically rotated in a region of φ2mm to φ4mm on the pupil. This region is a measurement region of the eye refractive power in the present example.

The light reception optical system 100b includes a hole mirror 114, a relay lens 120, a light reception diaphragm 121, a collimator lens 122, a ring lens 123, and an imaging element 124. The light reception optical system 100b extracts a reflection light flux of a measurement light flux reflected from the fundus in a ring shape via the peripheral portion of the pupil. The ring lens 123 is disposed at the pupil conjugate position, and the imaging element 124 is disposed at the fundus conjugate position. The eye refractive power is derived by analyzing the ring image formed on the imaging element 124 via the ring lens 123. The ring lens according to the present example includes double concentric lens portions, and the imaging element 124 receives double ring images. Of course, the ring lens 123 may be a single ring, or may be a triple or more ring.

As described above, in the present example, since the measurement light is eccentrically rotated at a high speed on the pupil, the analysis processing is performed on the output image from the imaging element 124 based on the exposure for a sufficiently long time with respect to the rotation cycle or the added image of the image data sequentially output from the imaging element 124, and the eye refractive power is derived. In the present example, values of SPH: spherical power, CYL: cylindrical power, and AXIS: astigmatic axis angle are acquired as a result of the analysis processing.

The measurement light flux from the measurement light source 111 passes through the relay lens 112, the hole portion of the hole mirror 114, and the prism 115, is reflected by each of the half mirror 116 and the half mirror 117, becomes coaxial with an optical axis L1, and further reaches the fundus via the objective lens 118. The reflection light flux obtained by reflecting the measurement light flux on the fundus passes through the optical path through which the measurement light flux has passed, is reflected by the mirror portion of the hole mirror 114, and reaches the imaging element 124 via the ring lens 123. The measurement optical system 100 is not limited to the above-described configuration, and may have a configuration in which the measurement light is projected to the subject eye after being formed into a ring shape, and the reflection light is captured as a pattern image using the imaging element 124.

### (Focus Adjustment Unit)

The focus adjustment unit 125 adjusts a focus position of the measurement optical system 100 (for example, the light reception optical system 100b) with respect to the subject eye E. The focus adjustment unit 125 includes a drive unit 126 such as a motor. The focus adjustment unit 125 can integrally move the measurement light source 111, the light reception diaphragm 121, the collimator lens 122, the ring lens 123, and the imaging element 124 of the measurement optical system 100 along an optical axis L4. For example, the focus adjustment unit 125 is moved according to the eye refractive power of the subject eye E, so that the measurement light source 111 and the imaging element 124 can be conjugate with the fundus. For example, when the subject eye is hyperopia, the imaging element 124 and the like are moved in a direction approaching the subject eye, and when the subject eye is myopia, the imaging element 124 and the like are moved in a direction away from the subject eye. At this time, the hole mirror 114 and the ring lens 123 are pupil conjugate at a constant magnification regardless of the movement of the focus adjustment unit 125. The movement position of the focus adjustment unit 125 is detected by the number of steps of a potentiometer or a motor.

### (Fixation Target Optical System)

The fixation target optical system 130 presents a fixation target to the subject eye E. The fixation target is presented on the optical axis of the measurement optical system 100. The fixation target optical system 130 is used to fixate the subject eye E. In addition, the fixation target optical system 130 is used to apply fogging and an accommodation load to the subject eye. For example, the fixation target optical system 130 includes at least a light source 131 and a fixation target plate 132. The fixation target plate 132 may be arranged at the fundus conjugate position. A fixation light flux from the light source 131 passes through the fixation target plate 132 on an optical axis L2, a lens 133, a lens 134, and the half mirror 116, and is reflected by the half mirror 117 to be coaxial with the optical axis L1. The fixation light flux further passes through the objective lens 118 and reaches the fundus.

### (Fixation Target Adjustment Unit)

The fixation target adjustment unit 135 can integrally move the light source 131 and the fixation target plate 132 of the fixation target optical system 130 along the optical axis. The fixation target adjustment unit includes a drive unit 136 such as a motor. The fixation target adjustment unit 135 can change a presentation distance of the fixation target plate 132 with respect to the subject eye E (that is, a presentation position of the fixation target) by moving the driver 135 according to the eye refractive power of the subject eye E, for example.

### (Observation Optical System)

The observation optical system 150 is used to capture a front image of an anterior segment of the subject eye E. For example, the observation optical system 150 includes an imaging element 151 and the like. The imaging element 151 may be arranged at the pupil conjugate position. An observation image of the anterior segment may be acquired as the front image. The observation image is used for alignment or the like. An index image (a point image and a Meyer ring image) projected onto the cornea from the index projection optical system 160 is captured by the observation optical system 150.

### (Index Projection Optical System)

The index projection optical system 160 is used to align (position) the measurement unit 3 with respect to the subject eye. The index projection optical system 160 includes, for example, a plurality of point light sources 161 and a ring light source 162. The point light source 161 projects an infinite target by irradiating the cornea with parallel light. The point light source 161 emits infrared light. The point light source 161 may emit visible light. The point light sources 161 are arranged vertically and horizontally symmetrically with respect to the optical axis L1. For example, in the present example, two point light sources are provided on each of the left and right sides. As a result, four point image indices are projected onto the cornea. The shape of the index is not limited thereto, and an index such as a line may be included. The number of indices is not limited thereto, and may be three or more point image indices. The ring light source 162 projects a finite distance index by irradiating the cornea with diffused light. The ring light source 162 emits infrared light. The ring light source 162 may emit visible light. The ring light source 162 is disposed in a ring shape around the optical axis L1. Thus, in the present example, a ring index (so-called Meyer ring) is projected onto the cornea. In the present example, a working distance adjustment may be performed by moving the measurement unit 3 in the front-rear direction so that the Purkinje image by the point light source 161 and the Meyer ring image by the ring light source 162 are captured at a predetermined ratio.

### [Control Unit]

The control unit 70 performs various controls of the present apparatus 1. The control unit 70 includes, for example, a general CPU (Central Processing Unit) 71, a ROM 72, a RAM 73, and the like. For example, the ROM 72 stores an ophthalmic apparatus control program for controlling the ophthalmic apparatus 1, initial values, and the like. For example, the RAM 73 temporarily stores various information. The control unit 70 is connected to each light source, each drive unit, each imaging element, the face support portion 4, the drive unit 5, the display unit 75, the operation unit 76, the storage unit (for example, nonvolatile memory) 74, and the like of the measurement unit 3. The storage unit 74 is, for example, a non-transitory storage medium capable of holding stored contents even when power supply is cut off. For example, a hard disk drive, a flash ROM, a detachable USB flash memory, or the like can be used as the storage unit 74.

### [Control Operation]

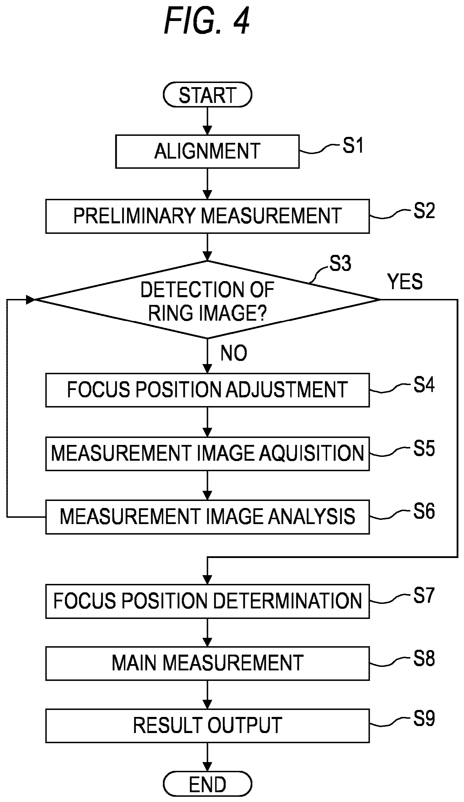

A control operation when the eye refractive power of the subject eye is measured in the ophthalmic apparatus 1 having the above configuration will be described with reference to the flowchart of FIG. 4.

### (Step S1: Alignment)

When the examinee causes the face support portion 4 to support the face according to an instruction from the examiner, the control unit 70 starts alignment. The control unit 70 starts presenting the fixation target and acquiring an anterior segment image. The control unit 70 adjusts the subject eye E and the ophthalmic apparatus 1 to a predetermined positional relationship based on an observation image of the anterior segment acquired by the observation optical system 150. More specifically, the control unit 70 performs alignment in the X and Y directions so that the optical axis L1 coincides with the corneal apex of the subject eye E based on an alignment index or the like detected in the observation image. The control unit 70 performs alignment in the Z direction so that the distance between the subject eye E and the ophthalmic apparatus 1 becomes a predetermined working distance.

### (Step S2: Preliminary Measurement)

When the alignment is completed, the control unit 70 starts preliminary measurement. In the preliminary measurement, the control unit 70 presents the fixation target by arranging the fixation target plate 132 at an initial position corresponding to a far point of a 0D (diopter) eye, which is an optically sufficient distance from the subject eye E. Further, the control unit 70 controls the focus adjustment unit 125 to arrange the focus position of the imaging element 124 (the measurement optical system 100) at a position corresponding to 0D, which is the initial position. The control unit 70 causes the light source 111 to emit the measurement light flux and causes the imaging element 124 to capture the reflection light flux of the measurement light flux as a ring image. The measurement image captured by the imaging element 124 is stored in the storage unit 74 or the like.

### (Step S3: Detection of Ring Image?)

The control unit 70 determines whether the ring image by the fundus reflection light can be detected from the measurement image acquired in the preliminary measurement. For example, the control unit 70 determines whether the ring image can be detected in each meridian direction by performing the edge detection processing on the measurement image stored in the storage unit 74. The position of the ring image may be detected by cutting the waveform of the luminance signal at a predetermined threshold value and obtaining the position from the midpoint of the waveform at the cutting position, the peak of the waveform of the luminance signal, the centroid position of the luminance signal, or the like. For the detection method of the ring image, refer to JP2012-075646A and the like.

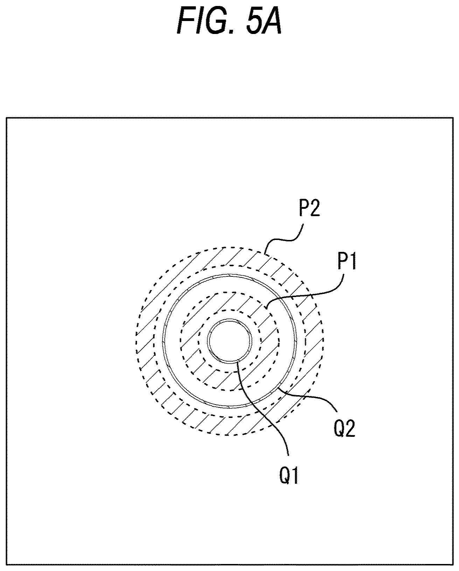

For example, as illustrated in FIG. 5A, when a ring image formed by fundus reflection light is blurred in a cataract eye or the like, the position of the ring image may not be detected. In such a case, the control unit 70 determines that the ring image cannot be detected, and proceeds to the process of step S4.

In the case of a cataract eye or the like, ring images Q1, Q2 (see FIG. 5A) by reflection (internal reflection) of an optical element (lens, prism, cover glass, or the like) is likely to be erroneously detected as ring images P1, P2 by fundus reflection light. Therefore, the control unit 70 determines whether the detected ring image is one caused by the fundus reflection or one caused by the internal reflection. In this case, the control unit 70 may determine whether the ring image is one caused by the fundus reflection or one caused by the internal reflection, based on the distiction information of the ring image due to internal reflection stored in the storage unit 74. The internal reflection is, for example, information such as the size, width, edge, chip, and luminance of the ring image.

For example, since a ring image formed by internal reflection light appears at a specific diopter, the diopter of the ring image formed by internal reflection (the size of the ring image corresponding to the focus position of the imaging element 124) may be stored as the internal reflection, and it may be determined that the ring image detected by the specific diopter has a high possibility of internal reflection.

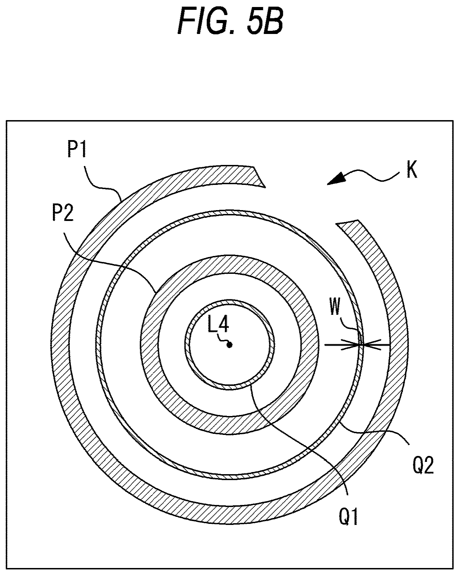

Further, since the width W (see FIG. 5B) of the ring image by the internal reflection light is narrower than that of the fundus reflection light, the width (the number of pixels) of the ring image may be stored as a threshold value as the internal reflection, and the ring image having the width less than the threshold value may be determined to have a high possibility of internal reflection.

In addition, since the edge of the ring image due to internal reflection is sharp, the rising (gradient strength) of the edge of the ring image due to internal reflection may be stored as the internal reflection, and it may be determined that the ring image having the edge equal to or greater than the threshold value has a high possibility of internal reflection.

In addition, since the fundus reflection light may be eclipsed by the iris, the ring image may be missing (see missing K in FIG. 5B), but since the internal reflection light is not eclipsed, it may be determined that the possibility of fundus reflection is high when the ring image is missing. When the double ring image is used as in the present example, the outer ring is more likely to be chipped, and thus the determination may be made in the state of the outer ring.

Even when the ring image due to the internal reflection is detected, the control unit 70 proceeds to step S4 assuming that the ring image due to the fundus reflection cannot be detected, and proceeds to the processing of step S7 when the ring image due to the fundus reflection can be detected.

### (Step S4: Focus Position Adjustment)

When it is determined that the ring image by the fundus reflection light cannot be detected from the measurement image, the control unit 70 controls the focus adjustment unit 125 to adjust the focus position of the imaging element 124 (the measurement optical system 100). For example, the control unit 70 causes the focus adjustment unit 125 to move the imaging element 124, the measurement light source 111, and the like in the optical axis direction, thereby adjusting the focus position to any position of +5D, -5D, -10D, -15D, and - 20D. Of course, the interval of the diopter may not be 5D, and may be every 1D, every 10D, or every arbitrary D. In addition, the intervals may not be equal.

### (Step S5: Measurement Image Acquisition)

When the adjustment of the focus position is completed, the control unit 70 acquires a measurement image based on the light reception signal of the imaging element 124. The acquired measurement image is stored in the storage unit 74 or the like.

### (Step S6: Measurement Image Analysis)

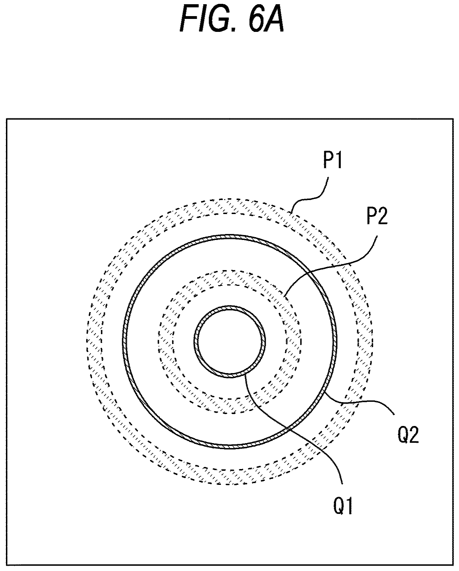

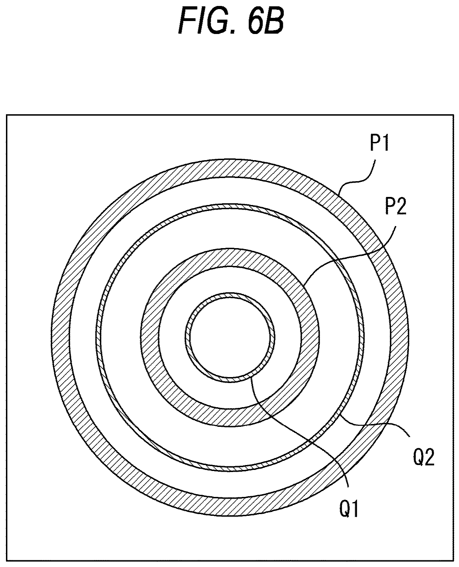

The control unit 70 analyzes the measurement image acquired after the adjustment of the focus position. As illustrated in FIG. 5A, even when the ring image of the fundus reflection light cannot be detected at the position of 0D due to cataract or the like, the focus position of the imaging element 124 is adjusted to the position corresponding to the refractive power of the subject eye, so that the ring image of the fundus reflection light is focused and the possibility that the ring image can be detected increases. For example, in the case of a cataract eye having a refractive power of about -10D, when the focus position is changed to a position of -5D (see FIG. 6A) and when the focus position is changed to a position of -10D (see FIG. 6B), the ring image of the fundus reflection light is focused and easily detected when the focus position is closer to the refractive power of the subject eye.

When the control unit 70 analyzes the measurement image, the control unit 70 returns to the processing of step S3 and determines whether or not the ring image by the fundus reflection light is detected. The control unit 70 repeats the processing of steps S3 to S6 until the ring image by the fundus reflection light can be detected. When the process of steps S3 to S6 is repeated, the control unit 70 adjusts the focus adjustment unit 125 to the focus position where the measurement image is not acquired. For example, when the focus position is shifted every 5D, the focus position is adjusted to a position where any of the measurement images of +5D, -5D, -10D, -15D, and -20D is not acquired, and the measurement image is acquired. The position of 0D has been acquired in the preliminary measurement of step S2. When the ring image by the fundus reflection light can be detected at any focus position, the control unit 70 proceeds to the processing of step S7.

In the example of FIG. 4, it is determined whether the ring image by the fundus reflection light is detected every time the focus position is adjusted and the measurement image is acquired and analyzed, but it is not limited thereto. For example, the control unit 70 may repeat the processing of steps S4 to S6 a plurality of times and then proceed to step S3. Further, the control unit 70 may proceed to step S3 after acquiring and analyzing the measurement images at all the focus positions for each 5D. In such a case, the control unit 70 may select the measurement image having the highest detection accuracy of the ring image by the fundus reflection light by comparing the plurality of measurement images.

### (Step S7: Focus Position Determination)

The control unit 70 calculates the eye refractive power based on the measurement image determined that the ring image can be detected by the preliminary measurement or the focus adjustment, and sets the position corresponding to the refractive power as the focus position at the time of the main measurement.

The calculation of the eye refractive power will be described. For example, when the subject eye E is an orthoscopic eye (that is, the spherical power is 0D), the reflection light flux from a fundus Ef is incident on the ring lens 123 as a parallel light flux (substantially parallel light flux). Therefore, a ring image having the same size as the ring lens 123 is formed on the imaging element 124. On the other hand, for example, when the subject eye E is a hyperopia (for example, the spherical power is +3D), a ring image enlarged according to the spherical power is formed on the imaging element 124. Further, for example, when the subject eye E is a myopia eye (for example, a spherical power is -3D or the like), a ring image reduced according to the spherical power is formed on the imaging element 124. For example, when the subject eye E is an astigmatic eye (for example, the cylindrical power is -2D and the astigmatic axis angle is 45 degrees), a ring image having an elliptical shape according to the cylindrical power and inclined according to the astigmatic axis angle is formed on the imaging element 124.

The control unit 70 thins the image data of the ring image and specifies the position of the ring image in each meridian direction. For example, the position of the ring image may be specified by detecting a luminance signal and obtaining a peak value, a centroid position, or the like thereof. Subsequently, the control unit 70 approximates the ring image by the least squares method or the like based on the specified position of the ring image, and derives the eye refractive power in each meridian direction from the shape of the approximated ring image.

When the focus position is adjusted to a position other than 0D in step S4, the eye refractive power is obtained by adding the adjusted diopter to the diopter obtained by analyzing the ring image. The focus position in the main measurement is determined based on the eye refractive power acquired as above.

### (Step S8: Main Measurement)

The control unit 70 controls the focus adjustment unit 125 to set the focus position determined in step S7, and performs the main measurement. Further, the control unit 70 may perform a fogging operation to release the accommodation of the subject eye.

When the fogging operation is performed, the control unit 70 moves the fixation target plate 132 to a fogging start position at which the subject eye E is in focus based on the eye refractive power of the subject eye E obtained in step S7. Thus, the fixation target is clearly observed by the subject eye E. Next, the control unit 70 moves the fixation target plate 132 to a fogging completion position corresponding to a predetermined fogging amount. At this time, the fixation target plate 132 is moved from the fogging start position toward the fogging completion position in a direction optically away from (away from) the subject eye E. When the fixation target plate 132 reaches the fogging completion position, the fogging of the subject eye E is completed. Accordingly, fogging is applied to the subject eye E, and the focus of the subject eye E is not aligned with the fixation target plate 132 again. The eye refractive power of the subject eye E approaches the true value from the eye refractive power obtained in the preliminary measurement, and the accommodation of the subject eye E is released.

The control unit 70 calculates the eye refractive power based on the measurement image acquired in a state where fogging is applied. The method of calculating the eye refractive power is the same as that in step S7. The control unit 70 calculates the final refractive power of the subject eye by summing the adjustment amount of the focus position and the diopter based on the analysis result of the ring image.

### (Step S10: Result Output)

The control unit 70 outputs the measurement result of the eye refractive power in the main measurement to a display unit, a printer, or the like. For example, the control unit 70 displays the eye refractive power (SPH, CYL, AXIS) of the subject eye E on the display unit.

As described above, by adjusting the focus position of the imaging element 124 by the focus adjustment unit 125, the measurable rate can be improved even when it is difficult to detect fundus reflection light due to cataract or the like. Further, as in the present example, by storing in advance the distinction information for distinguishing between the internal reflection light and the fundus reflection light, it is possible to suppress erroneous detection of the internal reflection light as the fundus reflection light.

Note that the control unit 70 may adjust the amount of light from the measurement light source 111 or the gain of the imaging element 124 in a case where the ring image by the fundus reflection light cannot be detected from the measurement image. Thus, the ring image may be easily detected. Further, the control unit 70 may adjust the amount of light from the measurement light source 111 or the gain of the imaging element 124 as the first stage process when the ring image cannot be detected, and may control the focus adjustment unit 125 to adjust the focus position of the imaging element 124 as the second stage process when the ring image cannot be detected again. In this way, the control unit 70 may shorten the measurement time as much as possible by performing control in a stepwise manner.

Since the position at which the internal reflection is detected is known by design, the control unit 70 may detect the internal reflection in the measurement image by moving the focus adjustment unit 125 to a predetermined position at which the internal reflection occurs. In this way, by specifying the ring image by the internal reflection light and excluding it from the detection candidate, the ring image by the fundus reflection light other than that may be easily detected. For example, when it is known that internal reflection occurs at positions (conjugate positions) corresponding to +25D, +4D, -5D, -16D, and -25D, the focus position may be adjusted to these positions to acquire the measurement image.

Although the focus adjustment unit 125 moves the imaging element 124 in the optical axis direction (the direction of the optical axes L1, L3 and L4), the present invention is not limited thereto. For example, the focus adjustment unit 125 may be configured to adjust the focus position of the imaging element 124 by inserting and removing an optical element such as a lens on the optical axis of the imaging element 124.

In the above example, the eye refractive power of the subject eye is measured by detecting the fundus reflection light from the subject eye as a ring image, but it may be detected as a pattern image of another shape. For example, the wavefront aberration of the subject eye may be measured by acquiring the fundus reflection light as a Shack-Hartmann image. Also in this case, whether the light is fundus reflection light or internal reflection light can be determined based on the brightness or sharpness of the Shack-Hartmann image.

### REFERENCE SIGNS LIST

- 1: Ophthalmic apparatus
- 4: Face support portion
- 3: Measurement unit
- 70: Control unit
- 75: Display unit
- 100: Measurement optical system
- 125: Focus adjustment unit

## Claims

1. An ophthalmic apparatus that measures an eye characteristic of a subject eye, the ophthalmic apparatus comprising:
a measurement optical system that includes a measurement light source and an imaging element, and measures the eye characteristic by capturing, as a pattern image, fundus reflection light of measurement light emitted from the measurement light source using the imaging element;
a focus adjustment unit that moves a focus position of the imaging element in an optical axis direction of the imaging element; and
a control unit that controls the focus adjustment unit to move the focus position, in a case where the pattern image is not detected from a measurement image acquired by the imaging element.

2. The ophthalmic apparatus according to claim 1, wherein
the control unit controls the focus adjustment unit to adjust the focus position, in a case where the pattern image is not detected from the measurement image after an amount of light from the measurement light source or a gain of the imaging element is adjusted.

3. The ophthalmic apparatus according to claim 1 or 2, further comprising:
a storage unit that stores in advance distinction information for distinguishing between a pattern image by the fundus reflection light and a pattern image by internal reflection light of the ophthalmic apparatus, wherein
the control unit determines whether the pattern image detected from the measurement image corresponds to the pattern image by the fundus reflection light or the pattern image by the internal reflection light, based on the distinction information.

4. The ophthalmic apparatus according to claim 3, wherein
the control unit moves the focus position to a predetermined position where a pattern image by the internal reflection light is detected, and excludes the pattern image by the internal reflection light detected at the predetermined position from a candidate for the pattern image by the fundus reflection light.

5. An ophthalmic apparatus control program executed in an ophthalmic apparatus that measures an eye characteristic of a subject eye, the ophthalmic apparatus control program comprising instructions which, when executed by a control unit of the ophthalmic apparatus, cause the ophthalmic apparatus to perform:
a measurement step of measuring the eye characteristic by capturing, as a pattern image, fundus reflection light of measurement light emitted from a measurement light source using an imaging element; and
a focus adjustment step of moving a focus position of the imaging element in an optical axis direction of the imaging element, in a case where the pattern image is not detected from a measurement image acquired by the imaging element.
